# EUROPÄISCHE PATENTSCHRIFT

(11) **EP 1 537 244 B1**
(45) Veröffentlichungstag und Bekanntmachung des Hinweises auf die Patenterteilung: **28.12.2011**
(21) Anmeldenummer: 03797279.1
(22) Anmeldetag: 08.09.2003
(51) Int. Cl.: C12Q 1/68

(54) **VERFAHREN ZUR DETEKTION VON BIOMOLEKÜLEN**
METHOD FOR DETECTING BIOMOLECULES
PROCEDE DE DETECTION DE MOLECULES BIOLOGIQUES

(30) Priorität: 13.09.2002 DE 10243303
(43) Veröffentlichungstag der Anmeldung: 08.06.2005
(73) Patentinhaber: Vuong, Giang Lam, 13353 Berlin (DE)
(72) Erfinder: Nordheim, Alfred, 72076 Tübingen (DE); Kammer, Winfried, 72076 Tübingen (DE); Weiss, Stefanie, 72076 Tübingen (DE); Vuong, Giang Lam, 13353 Berlin (DE)
(74) Vertreter: Patentanwälte Ruff, Wilhelm, Beier, Dauster & Partner
(86) Internationale Anmeldenummer: PCT/EP2003/009923
(87) Internationale Veröffentlichungsnummer: WO 2004/027091

(56) Entgegenhaltungen:
- US-A- 5 503 965
- US-A- 5 824 458
- RABILLOUD T: "A comparison between low background silver diammine and silver nitrate protein stains" ELECTROPHORESIS, Bd. 13, 1992, Seiten 429-439, XP001156152
- LUDÁNY A ET AL.: "Skimmed-milk blocking improves silver post-intensification of peroxidase-diaminobenzidine staining on nitrocellulose membrane immunoblotting" ELECTROPHORESIS, Bd. 14, 1993, Seiten 78-80, XP009020901
- M. LARSEN ET AL.: "Characterization of differently processed forms of enolase 2 from Saccharomyces cerevisiae by two-dimensional gel electrophoresis and mass spectrometry" ELECTROPHORESIS, Bd. 22, 2001, Seiten 566-575, XP002262909 ISSN: 0012-1797

## Beschreibung

Die Erfindung betrifft ein Verfahren zur Detektion von Biomolekülen mittels einer Silbar verbindung in Gegenwart mindestens eines mindestens bifunktionellen Agenses.

Die Detektion und Charakterisierung von Biomolekülen ist von fundamentaler Bedeutung für die biologische Forschung und die klinische Medizin. Insbesondere bei der Suche nach Mutationsereignissen sowie bei der Diagnostik genetisch bedingter Erkrankungen werden regelmäßig ,Detektions- und Charakterisierungsverfahren für unterschiedliche Biomoleküle eingesetzt.

Als Biomoleküle wird hier insbesondere die Gruppe, bestehend aus Peptiden, Proteinen, Glycoproteinen, Proteoglykanen, Kohlehydraten und Nukleinsäuren, verstanden.

Für den ersten Schritt der Detektion, die Auftrennung dieser Moleküle, werden derzeit zumeist ein- oder zweidimensionale Gelelektrophorese-Systeme eingesetzt. Elektrophorese bedeutet eine Auftrennung geladener Teilchen durch Einfluss eines elektrischen Feldes. Für die Elektrophorese können verschiedene Trägermaterialien eingesetzt werden, unter anderem Agarose-Gele, Zelluloseacetatgele oder Polyacrylamidgele. Aufgrund der im Vergleich zu Agarosegelen höheren Trennwirkung werden für die Proteincharakterisierung bevorzugt Polyacrylamidgele eingesetzt. Nach abgeschlossener gelelektrophoretischer Trennung müssen die Biomoleküle auf dem Trägermaterial sichtbar gemacht werden. Hierfür existieren verschiedene Visualisierungstechniken wie z. B. die Coomassie-Blau Färbung, Fluoreszenzmarkierung, radioaktive Markierung, Ethidiumbromidfärbung und Silberfärbung. Bei allen aufgeführten Methoden existieren große Unterschiede hinsichtlich der Sensitivität, des zeitlichen und materiellen Aufwandes, wie auch der Umweltverträglichkeit und Gesundheitsschädlichkeit der verwendeten Reagenzien bzw. der entstehenden Abfälle. So ist z. B. die Coomassie-Blau-Färbung sehr einfach durchzuführen, besitzt dafür allerdings eine sehr geringe Sensitivität. Vor allem bei der radioaktiven Markierung oder der Ethidiumbromidfärbung entstehen unerwünschte radioaktive bzw. kanzerogene Abfälle. Die Fluoreszenzmarkierung besitzt den Nachteil eines relativ großen apparativen Aufwandes. Die Silberfärbung ist gegenüber der Coomassie-Blau Färbung ca. einhundertfach sensitiver, es entstehen keine radioaktiven oder kanzerogenen Abfälle und der instrumentelle Aufwand ist relativ gering. Daher ist die Silberfärbung die derzeit am häufigsten eingesetzte Färbemethode zur Visualisierung von Proteinen. Die Silberfärbung besitzt jedoch den entscheidenden Nachteil, daß sie bei entsprechender erwünschter Sensitivität sehr zeitaufwendig ist. Dies ist jedoch vor allem in der modernen medizinischen Diagnostik und Life-Science-Forschung ein entscheidender Nachteil. Die verschiedenen Silberfärbemethoden lassen sich prinzipiell in zwei Gruppen, je nach verwendeter Silberverbindung, einteilen. Man unterscheidet zwischen Silbernitratfärbung und Silberdiaminfärbung. Näheres hierzu siehe Electrophoresis 13, 429-439 (1992) von T. Rabilloud. Eine Variante der Silbernitratfärbung ist and von Ludäny et al. in Electrophoresis 14, 78-80 (1993) beschrieben.

Ein weiterer Unterschied zwischen den unterschiedlichen Visualisierungstechniken sowie innerhalb der verschiedenen Variationen der Silberfärbung besteht in der Verfügbarkeit der Moleküle für weitere Charakterisierungsmethoden, insbesondere die massenspektrometrische Untersuchung. Bei vielen Visualisierungsmethoden bzw. bisherigen Silberfärbungen liegt das Molekül nach der Detektion in einer chemisch veränderten Form vor und steht daher einer massenspektrometrischen Untersuchung nicht mehr oder nur in einer für die Charakterisierung unzureichenden Form zur Verfügung.

Unabhängig von der exakten Färbemethode und der Verfahrensführung ist den meisten Silberfärbungen die Abfolge der Schritte Fixierung, Sensitivierung, Silberschritt (Inkubation mit einer Lösung, die Silberionen enthält), Entwickeln und Stoppen der Reaktion gemeinsam.

Zur Fixierung der Moleküle werden die Gele zunächst mit einer sauren alkoholischen Lösung inkubiert. Anschließend wird ein Sensitivierungsschritt durchgeführt, in welchem die Gele mit Reduktionsmitteln wie Glutaraldehyd, DTT, Dithionit oder Thiosulfat inkubiert werden. Diese sind für die Reduktion von Silberionen an der Oberfläche der Biomoleküle zu kleinsten Mengen an metallischem Silber verantwortlich und dienen im Entwicklungsschritt als Keimzelle für weiteren Niederschlag an Silber (Näheres hierzu siehe in Electrophoresis 11, 785-794 (1990) von T. Rabilloud). Nach einem Waschschritt, bei dem überschüssiges Reduktionsmittel entfernt wird, findet die Silberfärbung/Silberimprägnierung des Gels mittels Silbernitrat- bzw. Silberdiaminlösung statt. Nach diesem Silberschritt wird das Gel erneut gewaschen und anschließend mit einer Entwicklungslösung, die, entweder Formaldehyd und Natriumcarbonat oder Formaldehyd und Zitronensäure enthält, entwickelt. Nach abgeschlossener Entwicklung wird das Gel in einer Stoplösung inkubiert, um den Entwicklungsvorgang abzustoppen. Stoplösungen enthalten in der Regel Tris/Essigsäure, Zitronensäure oder Komplexbildner, wie beispielsweise EDTA oder EGTA.

Alle derzeit bekannten Methoden besitzen entweder den Nachteil, daß sie mit > 5 bis 24 h Silberfärbedauer sehr langwierig sind, um dabei eine relativ hohe Sensitivität zu erreichen, oder sie sind von vergleichsweise kurzer Dauer, d. h. < 5 h, aber dabei weniger sensitiv. Außerdem existieren relativ sensitive Silberfärbemethoden, die jedoch keine zufriedenstellende massenspektrometrische Untersuchung der Biomoleküle im Anschluss an die Detektion zulassen.

Wesentliches Ziel der Erfindung ist es, ein schnelles und gleichzeitig sehr sensitives Silberfärbeverfahren zu entwickeln, welches eine, sich an die Detektion anschließende, massenspektrometrische Charakterisierung der Biomoleküle zulässt.

Diese Aufgabe wird durch das erfindungsgemäße Detektionsverfahren mit den Merkmalen des Anspruchs 1 gelöst.

Erfindungsgemäß wird bei einem Verfahren zur Detektion von Biomolekülen, insbesondere von Peptiden, Proteinen, Glycoproteinen, Proteoglycanen, Kohlehydraten und/oder Nukleinsäuren, mittels einer Metallverbindung ein bifunktionelles Agens eingesetzt, welches einen hydrophoben und einen reduzierenden Teil besitzt. Es ist auch möglich, daß das Agens mehr als einen hydrophoben Teil und/oder mehr als einen reduzierenden Teil besitzt. Es ist ebenfalls denkbar, mehr als ein mindestens bifunktionelles Agens zur Detektion einzusetzen.

Gemäß der Erfindung handelt es sich bei dem bifunktionellen Agens um ein Molekül der allgemeinen Form X-R, wobei es sich bei dem Teil X des bifunktionellen Agens um einen Ascorbylrest und bei R um einen Hydrophoben Acyloxy- Acyl- oder Alkyl-Rest handelt.

In einer besonderen Ausführungsform handelt es sich bei R um einen gesättigten Kohlenwasserstoff. Es ist auch denkbar, daß es sich bei R um einen mindestens einfach ungesättigten Kohtenwasserstoff handelt. Gemäß einer bevorzugten Ausführungsform handelt es sich bei R um einen Acyloxy-Rest der allgemeinen Form -O-CO-CₙH(₂ₙ₊₁), wobei n vorzugsweise 8 - 21, insbesondere 11-17 und insbesondere bevorzugt 15, ist.

In einer besonderen Ausführungsform handelt es sich bei dem bifunktionellen Agens um Ascorbylpalmitat. Es ist jedoch auch denkbar, daß es sich um Ascorbylstearat, Ascorbylmyristat oder Ascorbyllaurat handelt.

Gemäß der Erfindung kann das bifunktionelle Agens während der Detektion in einer Endkonzentration von 10⁻⁵ bis 1 %, vorzugsweise von 10⁻⁴ bis 0,1 %, insbesondere 5 x 10⁻⁴ bis 5 x 10⁻³ %, vorliegen. In einer besonders bevorzugten Ausführungsform beträgt die Endkonzentration des bifunktionellen Agens 10⁻³ %.

Gemäß der vorliegenden Erfindung handelt es sich bei der Metallverbindung um eine Silberverbindung, insbesondere um Silbernitrat. In einer anderen Ausführungsform kann es sich bei der Silberverbindung auch um ein Silberdiamin handeln.

Bei den zu detektierenden Nukleinsäuren handelt es sich vorzugsweise um DNA oder RNA.

In einer Ausführungsform des erfindungsgemäßen Verfahrens werden die zu detektierenden Moleküle für die Detektion auf oder in einem Träger angebracht. Im Falle von Proteinen handelt es sich bei dem Träger vorzugsweise um ein Polyacrylamidgel. Im Falle von DNA handelt es sich bei dem Träger vorzugsweise um Agarosegele. Im Falle von RNA ist je nach Größe der RNA-Fragmente die Verwendung von Agarosegelen oder von Polyacrylamidgelen denkbar. In einer anderen Ausführungsform handelt es sich bei dem Trägermaterial um eine Membran, insbesondere eine PVDF- oder Nitrozellulose-Membran. Es ist auch denkbar, daß es sich bei dem Träger um einen Microarray-Träger, insbesondere um einen Biochip, handelt. Das erfindungsgemäße Verfahren kann auch zum Färben von Proteinen aus Zellen, welche mittels LCM (laser capture microdissection) aus einem Gewebe herausgelöst wurden, eingesetzt werden.

Gemäß der Erfindung umfasst das Verfahren zur Detektion der Biomoleküle mindestens die folgenden Schritte: Zunächst werden die Moleküle durch Inkubieren mit einer Fixierlösung auf oder in dem Träger fixiert, anschließend wird der Träger mit den Molekülen in mindestens einem Waschschritt mit einer ersten Waschlösung und anschließend mit einer zweiten Waschlösung gewaschen. Im anschließenden Metallverbindungsschritt wird das Trägermaterial mit den darauf oder darin fixierten Molekülen mit einer Lösung der Metallverbindung inkubiert und im anschließendem Waschschritt mit reinstem Wasser gewaschen. Hieran schließt sich der Entwicklungsschritt mit einer Entwicklungslösung und der abschließende Stopschritt an.

Nach dem erfindungsgemäßen Verfahren kann das bifunktionelle Agens im Fixierschritt, insbesondere als Zusatz zur Fixierlösung, eingesetzt werden. Die Fixierlösung kann neben dem bifunktionellen Agens 20 - 50 %, insbesondere 40 %, Ethanol enthalten.

Gemäß einer Ausführungsform wird das bifunktionelle Agens in einer mindestens teilweise alkoholischen Lösung eingesetzt. Vorzugsweise handelt es sich bei der alkoholischen Lösung um eine ethanolische Lösung, insbesondere aus absolutem Ethanol.

In dem erfindungsgemäßen Verfahren kann im Entwicklungsschritt ein Komplexbildner, insbesondere EDTA, als Bestandteil der Entwicklungslösung eingesetzt werden. In einer anderen Ausführungsform kann als Komplexbildner auch EGTA verwendet werden.

Als weitere Bestandteile kann die Entwicklungslösung neben dem Komplexbildner auch Natriumcarbonat, Natriumthiosulfat und/oder ein reduzierendes Reagens, vorzugsweise aus der Gruppe der Aldehyde, enthalten. In einer besonderen Ausführungsform der Erfindung handelt es sich bei dem reduzierenden Reagens um Formaldehyd.

Bei dem erfindungsgemäßen Verfahren kann nach der Detektion der Biomoleküle eine weitere Charakterisierung, vorzugsweise eine massenspektrometrische Untersuchung, insbesondere eine Identifizierung der Biomoleküle mittels MALDI-MS oder mit ESI-MS, durchgeführt werden.

Das erfindungsgemäße Verfahren lässt sich mit jedem Kit zur Detektion von Biomolekülen durchführen, welcher das Beschriebene bifunktionelle Agens enthält.

Im folgenden wird das erfindungsgemäße Verfahren durch ausführliche Beschreibung von besonderen Ausführungsformen sowie durch Figuren erläutert. In diesen Ausführungsformen können einzelne Merkmale der Erfindung allein oder in Kombination mit anderen Merkmalen verwirklicht sein. Die beschriebenen besonderen Ausführungsformen dienen lediglich zur Erläuterung und zum besseren Verständnis der Erfindung und sind in keiner Weise einschränkend zu verstehen.

Im übrigen ist die Erfindung anhand der Figuren dargestellt; dabei bedeuten:
Figur 1 zeigt die Sensitivität der Proteinfärbemethoden
Figur 2 zeigt ausgewählte Proteinspots für die massenspektrometrische Identifizierung am Beispiel eines mit kolloidalem Coomassie gefärbten 2D-Gels. Bei den anderen Färbungen wurden die korrespondierenden Spots für die MS-Identifizierung ausgewählt.
Figur 3 zeigt die MALDI-MS Sequenzabdeckung in %
Figur 4 (bestehend aus den Teilen 4A, 4B, und 4C) zeigt die Proteinidentifizierung mit ESI-MS nach tryptischem Verdau dargestellt in einer Tabelle bestehend aus den Teilen A, B und C.

### Figurenbeschreibung

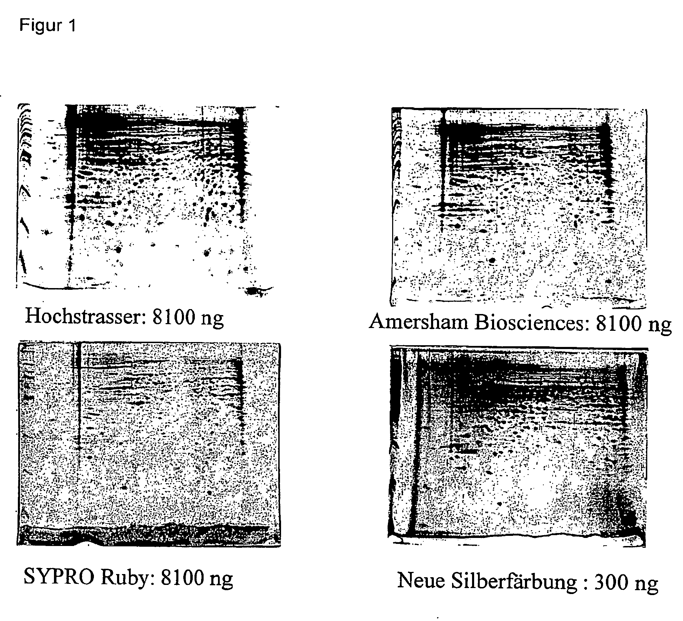

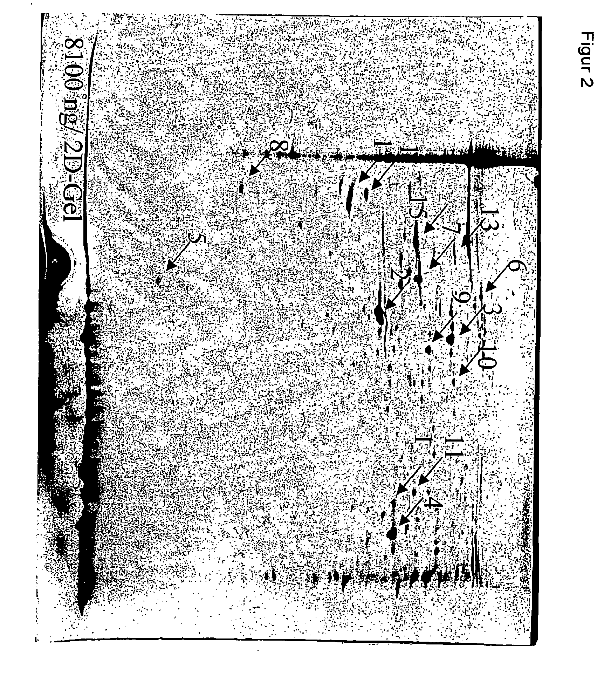

Figur 1 zeigt die unterschiedlichen Sensitivitäten der Proteinfärbung für die drei bekannten Proteinfärbemethoden nach Hochstrasser (siehe experimenteller Teil), Amersham Biosciences Plus One Silver Staining Kit (#17-1150-01) und dem Fluoreszenzfärbeverfahren SYPRO Ruby von Bio-Rad (# 170-3125), sowie das erfindungsgemäße neue Färbeverfahren. Das neue Verfahren zur Detektion von Biomolekülen ist mindestens um den Faktor 30 sensitiver als die bisher bekannten Methoden nach Hochstrasser und Amersham Biosciences und darüber hinaus deutlich sensitiver als die Markierung durch einen Fluoreszenzfarbstoff nach der Methode mit SYPRO Ruby. Die konventionellen Silberfärbemethoden, beispielsweise die Methode nach Hochstrasser oder Amersham Biosciences, besitzen zumeist den Nachteil, daß sie aufgrund der Verwendung von Glutaraldehyd als Sensitivierungsmittel im Sensitivierungsschritt die zu detektierenden Biomoleküle nach der Detektion einer anschließenden massenspektrometrischen Untersuchung nicht mehr oder nur unzureichend zugänglich sind. Das erfindungsgemäße Verfahren zur Detektion von Biomolekülen verwendet jedoch anstelle des Glutaraldehyds ein bifunktionelles Molekül, welches nicht die Nachteile des Glutaraldehyds besitzt und die Identifizierung der Biomoleküle durch Massenspektrometrie nach der Detektion ermöglicht. Zum Beweis dafür, daß Proteine nach erfolgter Detektion mit Hilfe des erfindungsgemäßen Verfahrensmittels massenspektrometrischer Methoden identifiziert werden können, wurde ein vergleichendes Experiment durchgeführt. Die in Figur 2 markierten 15 Proteinspots wurden aus vier 2-D-Gelen ausgewählt, die parallel mit vier unterschiedlichen Färbemethoden eingefärbt wurden. Bei den vier Färbemethoden handelte es sich erstens um die klassische kolloidale Coomassie-(G250)-Färbung, die als massenspektrometriekompatibel bekannt ist. Bei der zweiten Methode handelt es sich um die erfindungsgemäße Silberfärbemethode, welche im experimentellen Teil näher beschrieben ist. Bei der dritten Methode handelt es sich um die Färbung nach Hochstrasser unter Verwendung von Glutaraldehyd. Bei der vierten Methode handelt es sich um die Färbung mit dem Plus One Silver Staining Kit von Amersham Biosciences, ebenfalls unter Verwendung von Glutaraldehyd.

Im Anschluss an die unterschiedlichen Färbeverfahren wurden die einzelnen Proteinspots extrahiert und anschließend sowohl mittels MALDI-MS und ESI-MS untersucht. Die Ergebnisse dieser Proteinidentifizierungen sind in Tabelle 1 und Tabelle 2 dargestellt.

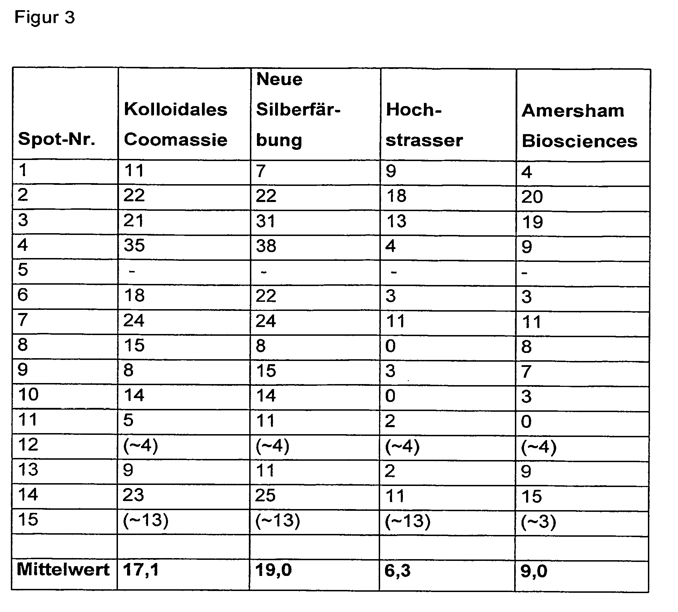

Figur 3 zeigt für die Spots Nr. 1 bis Nr. 15 die Sequenzabdeckung nach einer MALDI-MS in Prozent für die vier unterschiedlichen Proteinfärbemethoden. Es wurde die durchschnittliche Sequenzabdeckung für jede Methode bestimmt. Werte in Klammern wurden bei der Durchschnittsbildung nicht einbezogen, da die gefundenen Peptidmassen von verschiedenen Isoformen eines Proteins stammen könnten und so kein eindeutiger Wert für die Sequenzabdeckung bestimmbar ist. Bei dieser massenspektrometrischen Bestimmung erwies sich die erfindungsgemäße Silberfärbemethode mit 19 % Sequenzabdeckung sogar der klassischen Färbung mit kolloidalem Coomassie (17,1 % Sequenzabdeckung) als überlegen. Dies zeigt deutlich die Eignung des erfindungsgemäßen Detektionsverfahrens für eine im Anschluss an die Detektion durchgeführte massenspektrometrische Identifizierung der Biomoleküle. Die Ergebnisse der beiden Färbemethoden nach Hochstrasser (6,3 % Sequenzabdeckung) und der Färbung mittels des Kits von Amersham Biosciences (9 % Sequenzabdeckung) zeigen deutlich eine Beeinträchtigung der Identifizierbarkeit, durch, die auf die Verwendung von Glutaraldehyd zurückgeführt werden kann. Die insgesamt sehr niedrigen Sequenzabdeckungswerte resultieren aus den geringen Proteinmengen, welche für dieses vergleichende Experiment eingesetzt wurden.

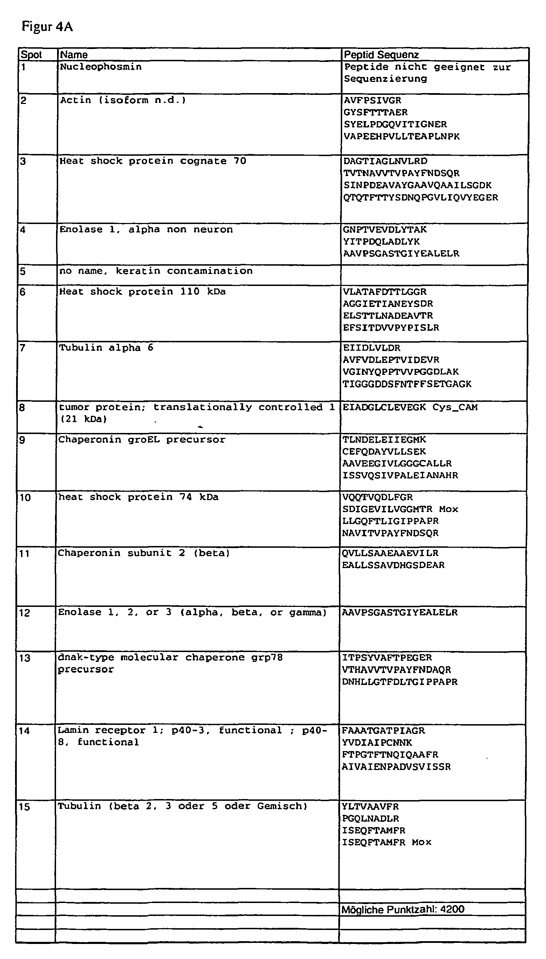

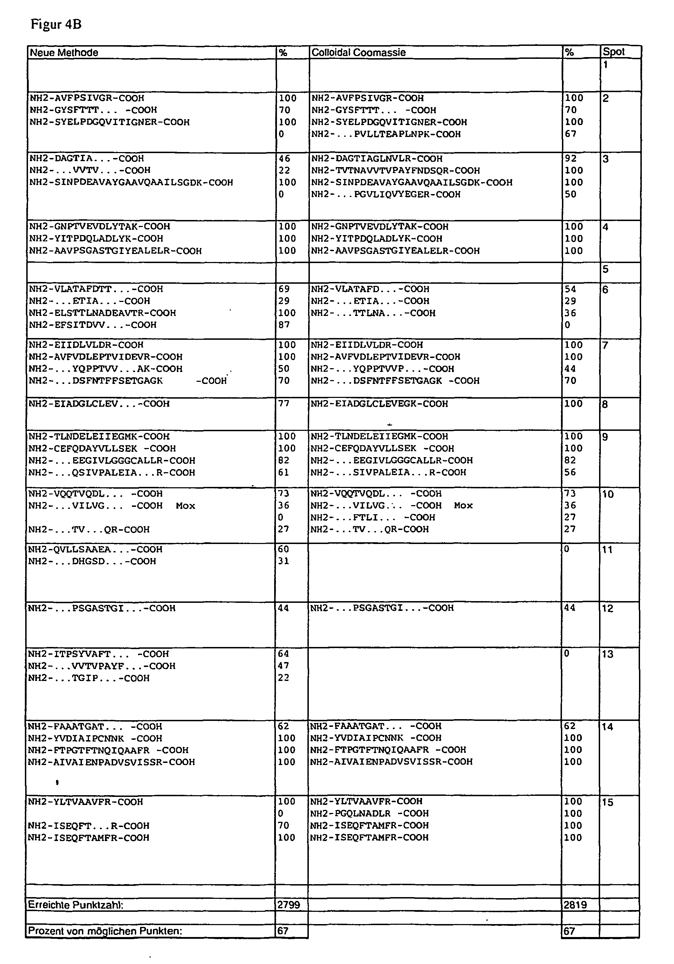

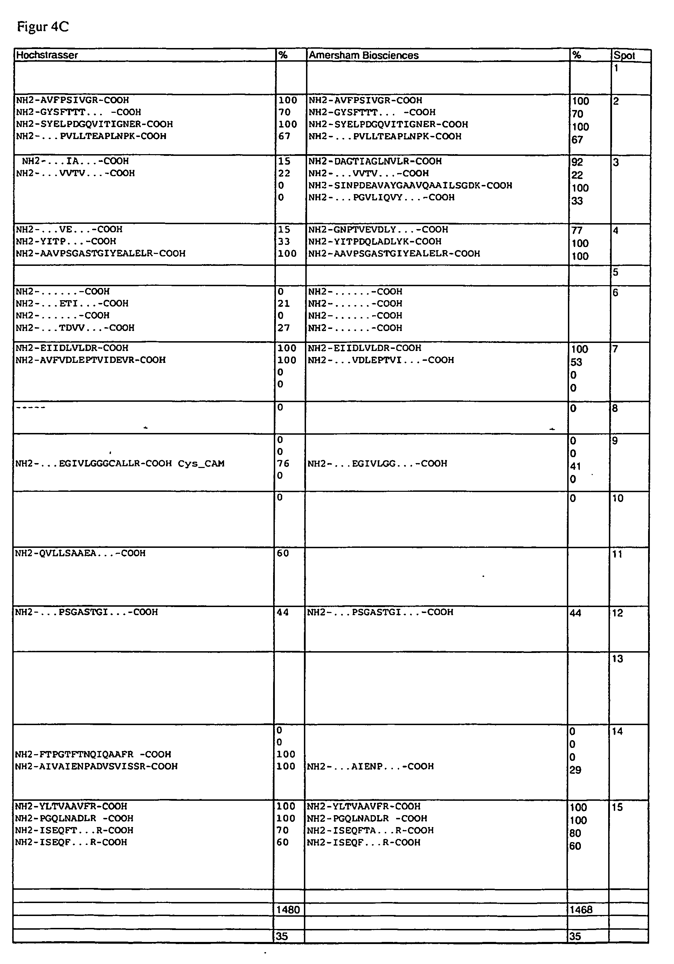

Figur 4 zeigt eine Darstellung der wiedergefundenen Aminosäuren von ausgewählten Peptiden aus den einzelnen Proteinspots und die Ermittlung der Sequenzabdeckung. Auch die Detektion mittels ESI-MS zeigt deutlich die Eignung des erfindungsgemäßen Detektionsverfahrens im Vergleich zur Methode nach Hochstrasser oder Amersham Biosciences. Die Sequenzabdeckung der neuen Silberfärbemethode betrug ebenso wie die der kolloidalen Coomassiefärbung 67 %, im Gegensatz zu 35 % bei den beiden anderen Methoden (Hochstrasser und Amersham Biosciences).

### Experimenteller Teil

Das für die unterschiedlichen Färbungen verwendete Proteingemisch wurde auf folgende Weise aus murinen embryonalen Stammzellen erhalten: 10 Mio. Zellen wurden in einem Eppendorf-Reaktionsgefäß zentrifugiert und das Pellet anschließend mit einem Lysepuffer, bestehend aus 9 M Harnstoff, 4 % CHAPS (Cholamidopropyldimethylammoniopropansulfonat), 1 % DTT (Dithio-threitol), 1 % Pharmalyte (pH 3-10) und 0,001 % Bromphenolblau lysiert. Die Konzentration der Proteinlösung wurde nach Bradford [Bradford, M. Analyt. Biochem. 72, 248-254, 1976] bestimmt.

### Silberfärbung von Biomolekülen:

### 1. Erfindungsgemäße Methode

Die zu färbenden Biomoleküle befinden sich in den Polyacrylamidgelen und werden während der ganzen Färbung auf einem Horizontalschüttler bewegt. Das Wechseln der Lösungen zwischen den einzelnen Färbeschritten kann durch Absaugen der nicht mehr benötigten und Zugabe frischer Lösungen oder durch Umsetzen der Gele in neue Färbeschalen erreicht werden.

### 1. Schritt: Fixieren der Biomoleküle

Das Polyacrylamidgel mit den darin befindlichen Biomolekülen wird in eine Fixierlösung, bestehend aus 40 %igem Ethanol sowie 10⁻³ % Ascorbylpalmitat, gegeben. Die Zugabe des Ascorbylpalmitats erfolgte in Form einer Lösung des Ascorbylpalmitats in absolutem Ethanol. Die Dauer der Fixierung beträgt 30 Minuten.

### 2. Waschschritte:

Die Gele werden zuerst mit einer 20 %igen und anschließend mit einer 10 %igen Ethanollösung gewaschen. Die Dauer beträgt jeweils 15 Minuten.

### 3. Silberschritt:

Nach Entfernen der Waschlösung werden die Gele in einer 0,5 %igen Silbernitratlösung für 30 Minuten inkubiert

### 4. Waschschritt:

Nach Entfernen der Silberlösung werden die Gele für 5 Minuten mit Milli-Q-Wasser gewaschen.

### 5. Entwicklung:

Die Waschlösung wird entfernt und die Gele werden mit der Entwicklungslösung für ca. 10-20 Minuten entwickelt, bis die gewünschte Intensität der Färbung erreicht ist. Die Entwicklungslösung besteht pro Liter aus 1,4 % Natriumcarbonat, 0,06 % EDTA, 240 µl 10 % Natriumthiosulfat-Lösung sowie 800 µl 37 %ige Formaldehydlösung.

### 6. Stoppen der Färbung:

Im Anschluss an die Entwicklung wird die Entwicklungslösung entfernt und durch eine Stoplösung ersetzt, welche aus 1,5 % EDTA-Lösung oder aus einer Lösung, bestehend aus 5 % Tris-Base und 2 % Essigsäure, gelegt. Die Dauer des Stopschrittes beträgt 5 Minuten.

### 2. Färbemethode nach Hochstrasser

Die zu färbenden Biomoleküle befinden sich auf Polyacrylamidgelen und werden während der ganzen Färbung auf einem Horizontalschüttler bewegt. Das Wechseln der Lösungen zwischen den einzelnen Färbeschritten kann man z. B. durch Absaugen der nicht mehr benötigten und Zugabe frischer Lösungen, oder durch Umsetzen der Gele in neue Färbeschalen erreichen
1. Fixierer 1 (40 % Ethanol + 10 % Essigsäure)
   Dauer der Fixierung: 1 h
2. Fixierer 2 (5% Ethanol + 5 % Essigsäure)
   Dauer der Fixierung: 2 h oder über Nacht
3. Waschschritt mit Milli-Q-Wasser
   Dauer: 5 Minuten
4. Fixierer 3 (74 g Natriumacetat-Trihydrat pro Liter + 20 ml 50 % Glutaraldehyd pro Liter)
   Dauer der Fixierung: 30 Minuten
5. 3 Waschschritte mit Milli-Q-Wasser
   Dauer: 3 mal 10 Minuten
6. Inkubation der Gele in Naphthalin-2,6-disulfonsäure
   Dauer: 30 Minuten
7. Inkubation der Gele in Naphthalin-2,6-disulfonsäure
   Dauer: 30 Minuten
8. 4 Waschschritte mit Milli-Q-Wasser
   Dauer: 4 mal 15 Minuten
9. Silberschritt
   Nach Entfernung der Waschlösung (Milli-Q-Wasser) werden die Gele für 30 Minuten in einer Silberdiaminlösung inkubiert.
   Die Silberdiaminlösung besteht aus 8 g Silbernitrat pro Liter, 13,3 ml 25 % Ammoniak und 4 ml 5 M NaOH.
10. 4 Waschritte mit Milli-Q-Wasser
   Dauer: 4 mal 4 Minuten
11. Entwicklungsschritt
   Die Waschlösung wird entfernt und die Gele werden mit der Entwicklerlösung für die Dauer von 5-10 min. entwickelt.
   Die Entwicklungslösung besteht aus 100 mg Zitronensäure und 1 ml 37 % Formaldehyd pro Liter
12. Stopschritt
   Die Entwicklerlösung wird entfernt und durch eine Stoplösung ersetzt.
   Die Stoplösung besteht aus 50 g Tris-Base mit 20 ml Essigsäure. Dauer: 5-10 Minuten

### 3. Amersham Biosciences Kit

Es wurde der Plus One Silver Stain Kit (Protein) verwendet und die Detektion entsprechend dem Herstellerprotokoll durchgeführt.

### 4. SYPRO Ruby

Für die Fluoreszenzmarkierung wurde der Kit SYPRO Ruby von Bio-Rad (# 170-3125) verwendet und die Detektion entsprechend dem Herstellerprotokoll mit einem Imagingsystem der Fa. Raytest (Fuji FLA 2000) durchgeführt.

### 5. Kolloidales Coomassie

Die zu färbenden Biomoleküle befinden sich in Polyacrylamidgelen und werden während der ganzen Färbung auf einem Horizontalschüttler bewegt. Das Wechseln der Lösungen zwischen den einzelnen Färbeschritten kann man z. B. durch Absaugen der nicht mehr benötigten und Zugabe frischer Lösungen oder durch Umsetzen der Gele in neue Färbeschalen erreichen

Die kolloidale Coomassie-Lösung besteht aus:
2 g Coomassie G250 in 1 I Milli-Q-Wasser gelöst + 55,5 ml 95-97 % Schwefelsäure. Die Lösung wird über Nacht gerührt und anschließend durch einen Filter filtriert. Anschließend werden 220 ml 10 M NaOH und 310 ml 100 % Trichloressigsäure zugegeben.

1. Fixierung und Färbung der Gele mit der Colloidalem Coomassie Lösung
   Dauer: über Nacht
2. Waschschritt mit Milli-Q-Wasser
   Die Färbelösung wird entfernt und mehrmals mit Milli-Q-Wasser gewaschen. Der Waschschritt wird solange durchgeführt, bis der Gelhintergrund auf ein Minimum reduziert ist.

## Patentansprüche

1. Verfahren zur Detektion von Molekülen, insbesondere von Peptiden, Proteinen, Kohlehydraten, Glycoproteinen, Proteoglykanen und/oder Nukleinsäuren mittels einer Silberverbindung in Gegenwart mindestens eines bifunktionellen Agens, bei dem es sich um ein Molekül mit der allgemeinen Formel X-R handelt, wobei es sich bei X um einen Ascorbylrest und bei R um einen hydrophoben Acyloxy-, Acyl- oder Alkyl-Rest handelt.

2. Verfahren nach Anspruch 1, dadurch-gekennzeichnet, daß es sich bei R um den Acyloxy-Rest der allgemeinen Form -O-CO-CₙH₍₂ₙ₊₁₎ mit n = 8-21, bevorzugt n = 11-17, insbesondere n = 15, handelt.

3. Verfahren nach Anspruch 1 oder Anspruch 2, **dadurch gekennzeichnet, daß** es sich bei dem bifunktionellen Agens um Ascorbylpalmitat (= Palmitoylascorbinsäure), Ascorbylstearat (= Stearoylascorbinsäure), Ascorbylmyristat (Myristoylascorbinsäure) oder Ascorbyllaurat (Lauroylascorbinsäure) handelt.

4. Verfahren nach einem der vorhergehenden Ansprüche, **dadurch gekennzeichnet, daß** es sich bei der Silberverbindung um Silbernitrat handelt.

5. Verfahren nach einem der vorhergehenden Ansprüche, **dadurch gekennzeichnet, daß** es sich bei den Nukleinsäuren um DNA oder RNA handelt.

6. Verfahren nach einem der vorhergehenden Ansprüche, **dadurch gekennzeichnet, daß** die Moleküle für die Detektion auf und/oder in einem Träger angebracht werden.

7. Verfahren nach Anspruch 6, **dadurch gekennzeichnet, daß** es sich bei dem Träger um ein Gel, insbesondere ein Polyacrylamid- oder Agarose-Gel, um eine Membran, insbesondere eine PVDF- oder Nitrocellulosemembran und/oder um einen Microarray-Träger, insbesondere einen Biochip, handelt.

8. Verfahren nach einem der vorhergehenden Ansprüche, **dadurch gekennzeichnet, daß** die Detektion der, insbesondere auf oder in dem Träger befindlichen, Moleküle mindestens folgende Schritte umfasst: Fixier-Schritt, mindestens einen Waschschritt, Metallverbindungs-Schritt, Entwicklungsschritt und/oder Stopschritt.

9. Verfahren nach Anspruch 8, **dadurch gekennzeichnet, daß** das bifunktionelle Agens im Fixierschritt eingesetzt wird und insbesondere in einer Fixierlösung enthalten ist.

10. Verfahren nach einem der vorhergehenden Ansprüche, **dadurch gekennzeichnet, daß** das bifunktionelle Agens in mindestens teilweise alkoholischer Lösung eingesetzt wird, vorzugsweise als Bestandteil der Fixierlösung, wobei es sich bei dem Alkohol vorzugsweise um Ethanol handelt.

11. Verfahren nach einem der Ansprüche 8 bis 10, **dadurch gekennzeichnet, daß** ein Komplexbildner, vorzugsweise EDTA und/oder EGTA, im Entwicklungsschritt eingesetzt wird, insbesondere in einer Entwicklungslösung enthalten ist.

12. Verfahren nach Anspruch 11, **dadurch gekennzeichnet, daß** die Entwicklungslösung ein reduzierendes Agens, bevorzugt aus der Gruppe der Aldehyde, insbesondere Formaldehyd, Natriumcarbonat, den Komplexbildner, und/oder Natriumthiosulfat enthält.

13. Verfahren nach einem der vorhergehenden Ansprüche, **dadurch gekennzeichnet, daß** nach der Detektion eine Charakterisierung, insbesondere eine massenspektrometrische Untersuchung, der detektierten Moleküle durchgeführt wird.

## Claims

1. A method for detecting molecules, in particular peptides, proteins, carbohydrates, glycoproteins, proteoglycans and/or nucleic acids, by means of a silver compound in the presence of at least one bifunctional agent, said agent being a molecule of the general formula X-R, wherein X is an ascorbic residue and R is a hydrophobic acyloxy, acyl or alkyl residue.

2. The method as claimed in claim 1, **characterized in that** R is an acyloxy residue of the general formula -O-CO-CₙH₍₂ₙ₊₁₎, where n = 8-21, preferably n = 11-17, in particular n = 15.

3. The method as claimed in claim 1 or in claim 2, **characterized in that** the bifunctional agent is ascorbyl palmitate (= palmitoyl ascorbic acid), ascorbyl stearate (= stearoyl ascorbic acid), ascorbyl myristate (myristoyl ascorbic acid) or ascorbyl laurate (lauroyl ascorbic acid).

4. The method as claimed in any of the preceding claims, **characterized in that** the silver compound is silver nitrate.

5. The method as claimed in any of the preceding claims, **characterized in that** the nucleic acids are DNA or RNA.

6. The method as claimed in any of the preceding claims, **characterized in that** the molecules are applied onto and/or into a support for detection.

7. The method as claimed in claim 6, **characterized in that** the support is a gel, in particular a polyacrylamide or agarose gel, a membrane, in particular a PVDF or nitrocellulose membrane, or a microarray support, in particular a biochip.

8. The method as claimed in any of the preceding claims, **characterized in that** the detection of the molecules, in particular those present on or in the support, comprises at least the following steps: fixing step, at least one washing step, metal compound step, developing step and/or stopping step.

9. The method as claimed in claim 8, **characterized in that** the bifunctional agent is used in the fixing step and, in particular, is present in a fixing solution.

10. The method as claimed in any of the preceding claims, **characterized in that** the bifunctional agent is used in an at least partially alcoholic solution, preferably as a component of the fixing solution, said alcohol preferably being ethanol.

11. The method as claimed in any of the claims 8 to 10, **characterized in that** a complexing agent, preferably EDTA and/or EGTA, is used in the developing step and, in particular, is present in a developing solution.

12. The method as claimed in claim 11, **characterized in that** the developing solution comprises a reducing agent, preferably from the group of aldehydes, in particular formaldehyde, sodium carbonate, the complexing agent and/or sodium thiosulfate.

13. The method as claimed in any of the preceding claims, **characterized in that** the detected molecules are **characterized, in** particular studied mass-spectrometrically, after detection.

## Revendications

1. Procédé pour la détection de molécules, en particulier de peptides, protéines, glucides (hydrates de carbone), glycoprotéines, protéoglycanes et/ou d'acides nucléiques, au moyen d'un composé à base d'argent en présence d'au moins un agent bifonctionnel, qui consiste en une molécule de formule générale X-R, X étant un radical ascorbyle et R étant un radical acyloxy, acyle ou alkyle hydrophobe.

2. Procédé selon la revendication 1, **caractérisé en ce que** R consiste en le radical acyloxy de formule générale -O-CO-CₙH₍₂ₙ₊₁₎ où n = 8-21, de préférence n = 11-17, en particulier n = 15.

3. Procédé selon la revendication 1 ou la revendication 2, **caractérisé en ce que** l'agent bifonctionnel consiste en palmitate d'ascorbyle (= acide palmitoylascorbique), stéarate d'ascorbyle (= acide stéaroylascorbique), myristate d'ascorbyle (= acide myristoylascorbique) ou laurate d'ascorbyle (= acide lauroylascorbique).

4. Procédé selon l'une quelconque des revendications précédentes, **caractérisé en ce que** le composé à base d'argent est le nitrate d'argent.

5. Procédé selon l'une quelconque des revendications précédentes, **caractérisé en ce que** les acides nucléiques consistent en ADN ou ARN.

6. Procédé selon l'une quelconque des revendications précédentes, **caractérisé en ce que** pour la détection on applique les molécules sur et/ou dans un support.

7. Procédé selon la revendication 6, **caractérisé en ce que** le support consiste en un gel, en particulier un gel de polyacrylamide ou d'agarose, en une membrane, en particulier une membrane de PVDF ou de nitrocellulose et/ou en un support de microréseau, en particulier une biopuce.

8. Procédé selon l'une quelconque des revendications précédentes, **caractérisé en ce que** la détection des molécules, en particulier se trouvant sur ou dans le support, comprend au moins les étapes suivantes : étape de fixage, au moins une étape de lavage, étape de composé de métal, étape de développement et/ou étape d'arrêt.

9. Procédé selon la revendication 8, **caractérisé en ce que** l'agent bifonctionnel est utilisé dans l'étape de fixage et en particulier est contenu dans une solution de fixage.

10. Procédé selon l'une quelconque des revendications précédentes, **caractérisé en ce que** l'agent bifonctionnel est utilisé en solution au moins partiellement alcoolique, de préférence en tant que composant de la solution de fixage, l'alcool étant de préférence l'éthanol.

11. Procédé selon l'une quelconque des revendications 8 à 10, **caractérisé en ce qu'**un complexant, de préférence l'EDTA et/ou l'EGTA, est utilisé dans l'étape de développement, en particulier est contenu dans une solution de développement.

12. Procédé selon la revendication 11, **caractérisé en ce que** la solution de développement contient un agent réducteur, de préférence choisi dans le groupe des aldéhydes, en particulier le formaldéhyde, du carbonate de sodium, le complexant et/ou du thiosulfate de sodium.

13. Procédé selon l'une quelconque des revendications précédentes, **caractérisé en ce qu'**après la détection on effectue une caractérisation, en particulier une analyse par spectrométrie de masse, des molécules détectées.
